(19) Europäisches Patentamt
European Patent Office
Office européen des brevets

(11) EP 4 146 617 B1

(12) # EUROPEAN PATENT SPECIFICATION

(45) Date of publication and mention of the grant of the patent:
**03.09.2025 Bulletin 2025/36**

(21) Application number: **21728346.4**

(22) Date of filing: **03.05.2021**

(51) International Patent Classification (IPC):
***C07C 51/02*** $^{(2006.01)}$ ***C07C 59/08*** $^{(2006.01)}$
***C07D 471/04*** $^{(2006.01)}$

(52) Cooperative Patent Classification (CPC):
(C-Sets available)
**C07D 471/04; C07C 51/02; C07C 59/08** (Cont.)

(86) International application number:
**PCT/US2021/030457**

(87) International publication number:
**WO 2021/225956 (11.11.2021 Gazette 2021/45)**

(54) **METHODS FOR SYNTHESIZING ANHYDROUS LACTIC ACID**

VERFAHREN ZUR SYNTHESE VON WASSERFREIER MILCHSÄURE

PROCÉDÉS DE SYNTHÈSE D'ACIDE LACTIQUE ANHYDRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.05.2020 US 202063019870 P**

(43) Date of publication of application:
**15.03.2023 Bulletin 2023/11**

(73) Proprietor: **Otsuka Pharmaceutical Co., Ltd.**
**Tokyo 101-8535 (JP)**

(72) Inventors:
- **BODHURI, Prabhudas**
  **Pleasanton, California 94588 (US)**
- **KHATRI, Hem Raj**
  **Pleasanton, California 94588 (US)**
- **DAVAR, Nipun**
  **Pleasanton, California 94588 (US)**

(74) Representative: **Marks & Clerk LLP**
**15 Fetter Lane**
**London EC4A 1BW (GB)**

(56) References cited:
**WO-A1-01/25180** **WO-A1-2015/092420**
**US-A- 2 331 948**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 51/02, C07C 59/08**

## Description

### FIELD

The present application and disclosed embodiments relate to improved methods, reactants and reagents for synthesizing lactic acid. More specifically, the present application and disclosed embodiments relate to improved methods, reactants and reagents for synthesizing anhydrous lactic acid.

### BACKGROUND

Lactic acid is a naturally occurring hydroxycarboxylic acid used in a variety of industries for a variety of applications. For example, lactic acid is used as a food additive, decontaminant and flavoring agent. Lactic acid is also a precursor for chemical and polymer synthesis of biodegradable and biocompatible polymers. In cosmetic and personal care products, lactic acid is used to moisturize skin, prevent acne and increase dermal collagen thickness. (Philipp, Babilas; Ulrich, Knie; Christoph, Abels, "Cosmetic and dermatologic use of alpha hydroxy acids" Journal of German Society of Dermatology, 10(7):488-49, 2012).

The global demand for lactic acid has increased steadily since 2013. In 2018, economic reports valued the global market for lactic acid at USD 2.64 billion and predicted an expected growth rate of 18.6% annually (CAGR) from 2019 to 2025. (Komesu, A., Oliveira, J. A. R. d., Martins, L. H. d. S., Wolf Maciel, M. R., and Maciel Filho, R., "Lactic acid production to purification: A review," BioResources. 12(2). 4364-4383, 2017); *see also* (Grand View Research, "Lactic Acid Market Size, Share & Trend Analysis Report" Report ID: 978-1-68038-126-9, 2019).

Lactic acid is chiral molecule consisting of two enantiomers.: L-(+)-lactic acid or (S)-lactic acid and its mirror image D-(-)-lactic acid or (R)-lactic acid. In the pharmaceutical industry, the purity and chemical structure of reactants and reagents can drastically affect the resulting properties and therapeutic effectiveness of active pharmaceutical ingredients (APIs). There are very few suppliers for high quality lactic acid and specifically anhydrous lactic acid suitable in the manufacture of APIs. Most suppliers provide lactic acid in about 80%-95% solution with water (e.g., about 90% solution with water). Aqueous lactic acid contains significant impurities that are not suitable in the manufacture of APIs.

The primary methods of preparing L-lactic acid involve fermentation of carbohydrates followed by recovery and purification. Some of these methods are described in U.S. Patent No. 2,232,554 and other published literature. (Borsook, H., Huffinan, H.M. and Liu, Y.-P., J. Biol. Chem., "The Preparation of Crystalline Lactic Acid," 102, 449, 1933). For example, a lactic acid solution can be produced by converting crude lactic acid into Ca-lactate by fermentation, crystallizing Ca-lactate, neutralizing Ca-lactate with mineral acid (sulfuric acid), and filtering to obtain a lactic acid solution. (Sidney Hsin-Huai Chow "Lactic Acid Review," A Master's Thesis, 1957). Other methods of producing lactic acid involve fractional distillation of mixed ethers (ethyl and isopropyl ethers) in greater than 50% commercial syrup followed by crystallization. (Borsook, H., Huffinan, H.M. and Liu, Y.-P., J. Biol. Chem., "The Preparation of Crystalline Lactic Acid," 102, 449, 1933). These solvent extraction methods used to extract lactic acid from aqueous solution suffer from low efficiency. PCT/EP2015/067258 describes the conversion of crude lactic acid into a solid Mg-lactate followed by acidification with hydrochloric acid gas to produce a lactic acid solution from which lactic acid can be recovered by employing additional isolation and purification steps.

The preparation of anhydrous lactic acid often requires strenuous and complex post fermentation steps, which commonly include derivatization to Ca-/Zn-/$NH_4$- salt, isolation of the respective salts, acid treatment of the salts, distillation, crystallization and additional isolation steps. One such method utilizes distillation at reduced pressure to obtain monomeric lactic acid, which is subjected to adiabatic crystallization to obtain dry lactic acid crystals. *See e.g.,* U.S. Patent No. 6,630,603; EP Patent No. 1317408; U.S. Patent Publication No. 2011/0319660.

There are no known and reliable processes available to isolate anhydrous lactic acid directly into non-aqueous solvents without involving aqueous lactic acid solution. There are also no known processes for the chemical synthesis of anhydrous lactic acid at industrial scales without employing a complex number of inefficient steps that ultimately result in an aqueous or low purity product. Therefore, improved methods for synthesizing anhydrous lactic acid are necessary to address the deficiencies in purity, complexity and yields of available conventional processes.

WO 01/25180 A1 relates to a process for producing a purified lactic acid solution. US 2 331 948 A relates to a method for the purification of lactic acid. WO 2015/092420 relates to bicyclic heterocycle compounds and their uses in therapy.

### SUMMARY

The present application and disclosed embodiments are directed to improved methods, reactants and reagents for synthesizing lactic acid on industrial scale for all applications. More specially, the present application and disclosed embodiments are directed to improved methods, reactants and reagents for synthesizing anhydrous lactic acid for

pharmaceutical applications.

The present invention is defined as set out in the claims.

The foregoing and other objects, features and advantages of the present disclosure will become more readily apparent from the following detailed description of exemplary embodiments as disclosed herein.

## BRIEF DESCRIPTION OF THE DRAWINGS

Embodiments of the present application are described, by way of example only, with reference to the attached Figures, wherein:

FIG. 1 depicts an exemplary reaction scheme for the synthesis of anhydrous lactic acid;
FIG. 2 depicts anhydrous lactic acid crystals produced with an exemplary synthesis scheme;
FIG. 3 depicts an exemplary equipment layout for the synthesis of anhydrous lactic acid; and
FIG. 4 depicts $^1$HNMR results for an L-(+)-Lactic Acid end-product synthesized using an exemplary synthesis scheme.
FIG. 5 depicts a representative chromatogram of a standard solution of D- and L- lactic acid using a chiral HPLC protocol described herein.

## DETAILED DESCRIPTION

The following examples and embodiments disclosed and described in this application are illustrative. Those of ordinary skill in the art will understand that various alterations to the embodiments may exist without departing from the scope or intent of the present application or exemplary embodiments disclosed, including variations with respect to the synthesis methods, processes, reactants, reagents, parameters and conditions described herein. The present application is directed to improved methods, reactants and reagents for synthesizing anhydrous lactic acid for use in industrial processes, including but not limited to, petrochemical processes; chemical and polymer synthesis; processes for the production of cosmetics and personal care products; processes for the production of food additives, decontaminants and flavoring agents; and processes for the production of pharmaceuticals and active pharmaceutical ingredients.

In an exemplary embodiment, an anhydrous lactic acid compound of formula (I) is produced by the synthesis and process steps depicted in FIG. 1. The exemplary synthesis of FIG. 1 can be used to produce anhydrous lactic acid, and specifically L-(+)-lactic acid, D-(-)-lactic acid and DL-lactic acid.

For the synthesis of anhydrous lactic acid depicted in FIG. 1, n is an integer other than 0 and x is 0, or an integer other than 0. In an exemplary embodiment, n is 1 or 2 and x is 0 to 6.

M is an alkali metal or alkaline earth metal. Suitable alkali metals and alkaline earth metals include, but are not limited to, calcium, sodium, potassium, magnesium, lithium, cesium, barium, beryllium or strontium. In an exemplary embodiment, M is calcium and the compound of formula (Ia) is calcium lactate hydrate. In another exemplary embodiment, the compound of formula (Ia) is calcium L-lactate pentahydrate.

$H_nX$ is an acid. Suitable mineral acids include, but are not limited to, sulfuric acid, hydrochloric acid, hydrobromic acid, hydroiodic acid, perchloric acid, chloric acid, hydrofluoric acid and nitric acid. Suitable organic acids include, but are not limited to, malonic acid, glutaric acid, citric acid, malic acid, tartaric acid, oxalic acid or formic acid. In an exemplary embodiment, the acid is sulfuric acid or oxalic acid.

X is the conjugate base of the acid used in the synthesis of FIG. 1. Depending on the acid used in the synthesis of FIG. 1, the conjugate base can vary. Suitable examples of conjugate bases include, but are not limited to, $HSO_4^-$, $Cl^-$, $Br^-$, $F^-$, $I^-$, $ClO_4^-$, $ClO_3^-$, $NO_3^-$, $C_2O_2^{4-}$, $C_3H_3O_4^-$, $C_5H_6O_4^{2-}$, $C_6H_5O_7^{3-}$, $C_4H_5O_5^-$, $C_4H_4O_6^{2-}$, $H_2PO_4^-$ or $HCO_2^-$.

In an exemplary step of the synthesis of FIG. 1, the compound of formula (Ia) is reacted with an acid compound of formula $H_nX$ in a first solvent.

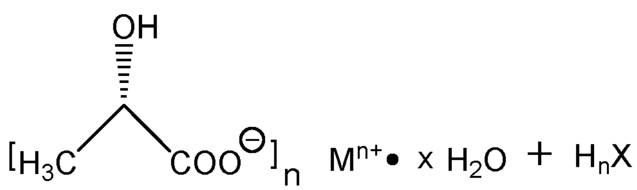

(Ia)

The first solvent can be one or more ester solvents, including but not limited to, methyl acetate, ethyl acetate, isopropyl acetate or n-propyl acetate. Other suitable solvents include acetone, toluene, acetonitrile, methyl tert-butyl ether

(MTBE), isopropanol, ethanol, methanol, tetrahydrofuran, butanol, butyl acetate, carbon tetrachloride, dichloroethane, dichloromethane, diethyl ether, diisopropyl ether, dimethyl sulfoxide, heptane hexane, methyl acetate, ethyl ketone, methyl tert-butyl ether, or methyl isobutyl ketone. The first solvent can be selected based on the ultimate use of final anhydrous lactic acid product. In exemplary embodiments, the first solvent is methyl acetate, ethyl acetate, isopropyl acetate and/or toluene.

The reaction of the compound of formula (Ia) with the acid compound of formula $H_nX$ in the first solvent produces a reaction mixture containing the compound of formula (Ib) and lactic acid (I) in solution with the first solvent and/or water.

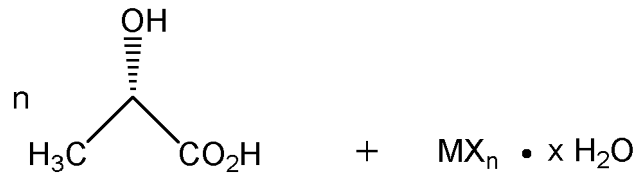

(I) (lactic acid in solution) (Ib)

Each n described herein is independently an integer other than 0. By way of example only, when the acid is HCl, n in $H_nX$ is 1; when the acid is sulfuric acid, n in $H_nX$ is 2. By way of example only, when calcium-lactate hydrate is used as Formula (Ia) reacting with HCl, n is 2 in $M^{n+}$, n is 1 in $H_nX$, and n is 2 in

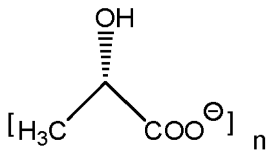.

By way of example only, when calcium chloride is used in the compound of Formula (Ib) n is 2 in $MX_n$. In some embodiments, each n in the equation above has the same value based on stoichiometry. The stoichiometry and/or the value for each n will be readily apparent to one of skill in the art.

In one exemplary embodiment, the compound of formula (Ib) is at least partially insoluble in the first solvent, and lactic acid (I) is at least partially soluble in the first solvent. In one exemplary embodiment, the compound of formula (Ib) is insoluble in the first solvent, and lactic acid (I) is soluble in the first solvent.

In the next step of the synthesis of FIG. 1, the reaction mixture containing the lactic acid (I) in solution with the first solvent and the compound of formula (Ib) is filtered to concentrate a first filtrate containing lactic acid (I) in solution with the first solvent and/or water. The first filtrate containing lactic acid can be further processed, isolated, recovered, filtered, concentrated, distilled, decanted, crystallized, dried and/or dehydrated to produce anhydrous lactic acid.

Suitable filtration techniques include vacuum filtration, gravity filtration, cold filtration, hot filtration, Nutsche agitated filtration, centrifuge filtration and robo-filtration. In an exemplary embodiment, gravity filtration is performed using a Buchner funnel. In another exemplary embodiment, gravity filtration is performed using a Hirsch funnel. The Buchner funnel and Hirsch funnel can be equipped with filter paper and cloth to facilitate the filtration.

In an exemplary embodiment after the first filtration, the first filtrate containing lactic acid in solution with the first solvent and/or water can be distilled to remove water and/or the first solvent and concentrate the lactic acid in the first filtrate. Maximizing the removal of the first solvent and water from the first filtrate by distillation maximizes the yield of anhydrous lactic acid.

After the initial filtration and distillation steps, the first filtrate containing lactic acid (I) can be isolated and subsequently crystallized by combining the lactic acid (I) with a second crystallization solvent and cooling the solution to create purified crystals of anhydrous L-lactic acid (I). Optionally, seed crystals of lactic acid can be added to the solution to initiate and accelerate crystallization.

To maximize recovery and yield of the end-product, lactic acid is only partially soluble in the second crystallization solvent. The second crystallization solvent can be one or more solvents including but not limited to toluene and heptane. Additional filtration steps can be employed to isolate and purify the resulting anhydrous lactic acid product.

Other processing steps can be employed after the first filtration step to increase the yield of anhydrous lactic acid. For example, residual water can be removed from the isolated first filtrate by azeotropically removing the water to produce anhydrous lactic acid. In an exemplary embodiment, azeotropic removal can be achieved by azeotropic distillation. A distillation solvent, such as toluene can be used to create an azeotrope with water to effect azeotropic distillation. In an exemplary embodiment, a Dean-Stark water trap can be used to azeotropically remove water from the isolated first filtrate containing lactic acid in solution to produce a dry/anhydrous lactic acid.

The degree of crystallization and purification of anhydrous lactic acid obtained from the solution containing crystallization solvent and lactic acid is heavily dependent on the water content of the filtrate and elimination of water from the first filtrate produced after initial filtration. In addition to the exemplary steps of synthesis disclosed herein, water content of the filtrate can be monitored using Karl Fischer titration and residual water can be azeotropically removed as described herein to increase yield and purity of the anhydrous lactic acid product. In an exemplary embodiment, water can also be removed by drying the filtrate over a drying agent, such as anhydrous $Na_2SO_4$. Molecular sieves and drying ovens can also be used to remove water from the first filtrate.

The exemplary methods of synthesizing anhydrous lactic acid disclosed herein can produce an anhydrous lactic acid product with less than 0.1% by weight water and less than 1% by weight polylactate impurities, such as lactide, oligomers of lactic acid and mixtures thereof. In some embodiments, the resulting anhydrous lactic acid is essentially free of water and free of polylactic acid and can be used in high precision, high purity industrial processes, such as the production of APIs. The resulting anhydrous lactic acid is substantially free of water and substantially free of polylactic acid and can be used in high precision, high purity industrial processes, such as the production of APIs. As used herein "substantially free of water and substantially free of polylactic acid" means that the product has less than about 5%, about 4%, about 3%, about 2%, about 1% or about 0.5% of water and/or polylactic acid.

**In** an exemplary embodiment of the synthesis of anhydrous lactic acid depicted in FIG. 1, calcium lactate hydrate can be neutralized with concentrated sulfuric acid in methyl acetate using Reaction Scheme 1.

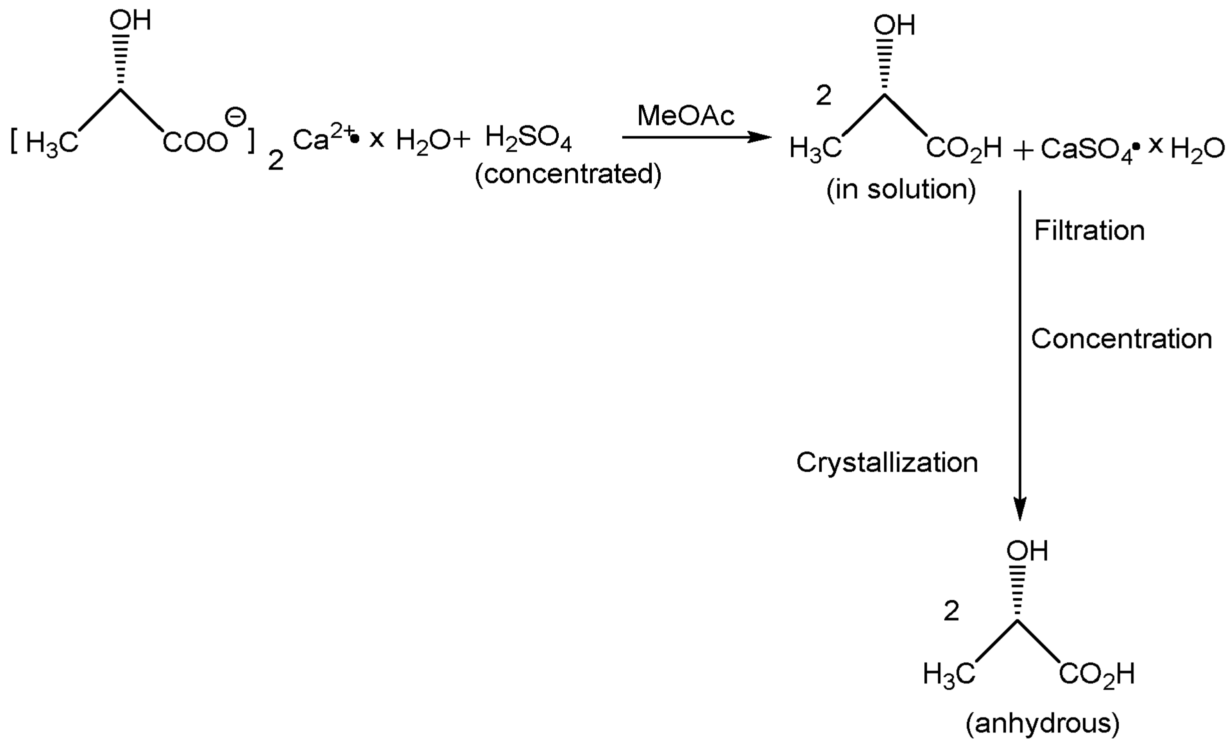


**Reaction Scheme 1**

The calcium lactate hydrate used in Reaction Scheme 1 and methyl acetate (MeOAc) was selected as a solvent based on the ultimate use of the anhydrous lactic acid end-product in the synthesis of an active pharmaceutical ingredient (API). After reacting calcium lactate hydrate with concentrated sulfuric acid in methyl acetate, the resulting slurry containing lactic acid can be filtered and concentrated. Residual water can be removed by absorption with calcium sulfate and additionally with azeotropic removal of residual water using vacuum distillation, including rotary evaporators or direct vacuum distillation, to produce an anhydrous lactic acid end-product.

In an exemplary embodiment of the synthesis of anhydrous lactic acid of FIG. 1, calcium lactate hydrate can be neutralized with hydrochloric acid in methyl acetate using Reaction Scheme 2.

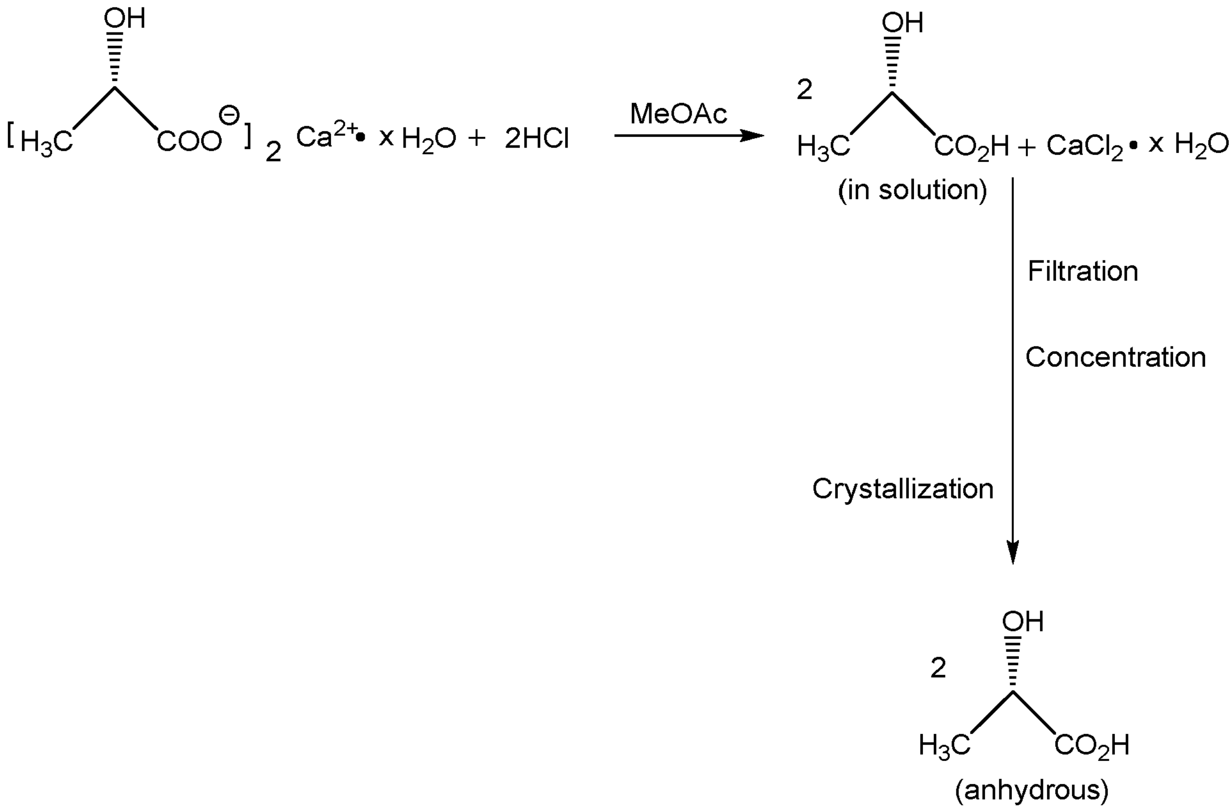


**Reaction Scheme 2**

After reacting calcium lactate hydrate with hydrochloric acid, the calcium chloride produced forms a hydrated insoluble in MeOAc, which can be filtered off. The filtrate containing lactic acid can be further processed by isolating, recovering, filtering, concentrating, decanting distilling, crystallizing and/or dehydrating the filtrate to produce high purity anhydrous lactic acid.

In an exemplary embodiment of the synthesis of anhydrous lactic acid of FIG. 1, calcium lactate hydrate can be neutralized with oxalic acid in methyl acetate using Reaction Scheme 3 below.

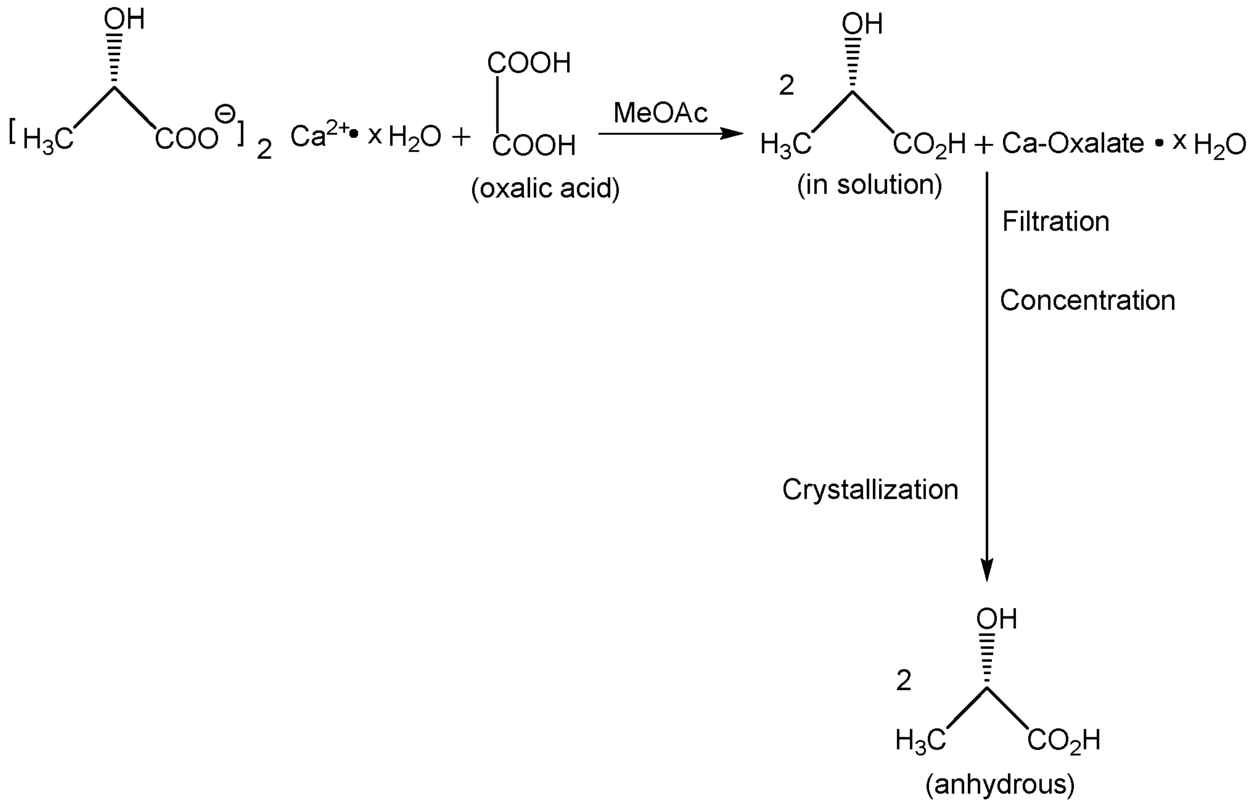


**Reaction Scheme 3**

Calcium lactate hydrate can be neutralized with oxalic acid, which has pKa less than the pKa of lactic acid. Reaction Scheme 3 utilizes a slurry to slurry reaction in methyl acetate. Calcium oxalate is insoluble in methyl acetate and can be filtered off to create a pure lactic acid filtrate. The pure lactic acid filtrate can be further processed by isolating, recovering, filtering, concentrating, decanting, distilling, crystallizing and/or dehydrating the filtrate to produce high purity anhydrous lactic acid.

The anhydrous lactic acid end-product produced by Reaction Schemes 1-3 can be used to produce the API compound of formula (XXIII) by reacting the compound of formula (XXII), i.e., 1-{6-[( 4-fluorophenyl)methyl]-5-(hydroxymethyl)-3,3-dimethyl-1H,2H,3H-pyrrolo[3,2-b ]pyridin-l -yl}-2-[(2R,5R)-5-methyl-2-{[(3R)-3-methylmorpholin-4-yl] methyl}piperazin-l-yl]ethan-1-one (IUPAC name) or 1-(6-(4-fluorobenzyl)-5-(hydroxymethyl)-3,3-dimethyl-2,3-dihydro-1H-pyrrolo[3,2-b]pyridin-1-yl)-2-((2R,5R)-5-methyl-2-(((R)-3-methylmorpholino)methyl)piperazin-1-yl)ethan-1-one (name generated from ChemDraw Professional V. 17.1.0.105 (19), with anhydrous lactic acid in a solution of ethyl acetate as shown in the reaction step below.

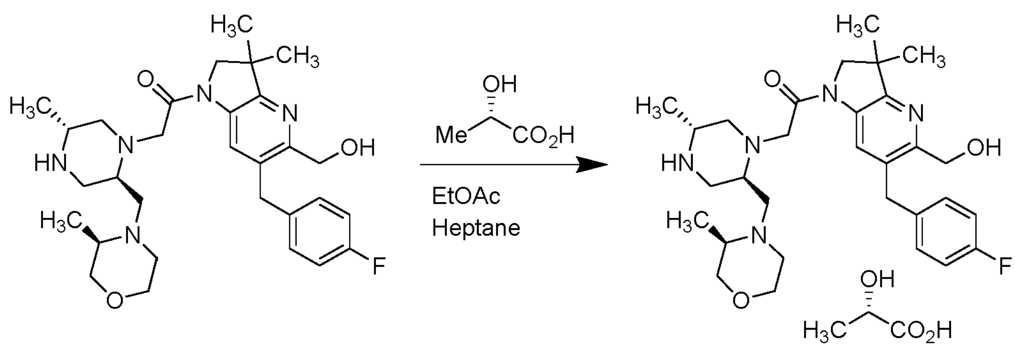


(XXII) (XXIII)

Accordingly, provided herein is a method the preparation of a compound of Formula (XXIII):

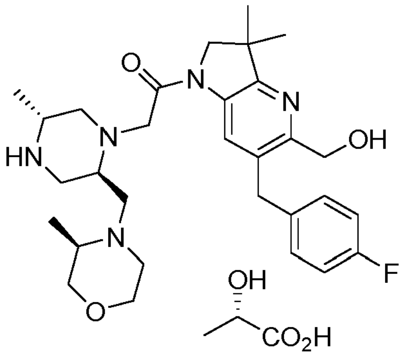

(XXIII)

the method comprising contacting a compound of Formula (XXII)

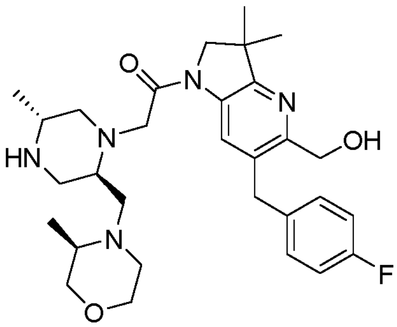

(XXII)

with the anhydrous lactic acid described herein to provide the compound of Formula (XXIII).

The examples below describe exemplary reaction conditions, parameters and reagents to carry out exemplary steps in the synthesis of anhydrous lactic acid. The following examples are illustrative of some of the embodiments described herein. Those of ordinary skill in the art will understand that various alterations to the examples may exist without departing from the scope or intent of the present application or exemplary embodiments disclosed, including variations with respect to the synthesis methods, processes, reactants, reagents, parameters and conditions described herein. The examples below can produce an anhydrous lactic acid product with less than 0.1% by weight water and less than 1% by weight polylactate impurities, such as lactide, oligomers of lactic acid and mixtures thereof. The examples can also be used to produce anhydrous lactic acid, and specifically L-(+)-lactic acid, D-(-)-lactic acid and DL-lactic acid.

**Abbreviations**

| | |
|---|---|
| AcOH | Acetic acid |
| EtOAc | Ethyl acetate |
| h | hours |
| IPAC or iPrOAc | Isopropyl acetate |
| min | minutes |
| Me | Methyl |
| MeOAc | Methyl acetate |
| ROI | Residue on ignition |
| Tol | toluene |

**EXAMPLES**

**GENERAL PROCEDURES:**

The Examples described below may be used for preparation of high purity anhydrous lactic acid, including L-(+)-lactic acid, D-(-)-lactic acid and/or DL-lactic acid. The following examples are for the purpose of illustration only and are not meant to limit the scope of the embodiments and/or claims herein.

Chiral purity of the product was measured by an isocratic HPLC method using a chiral column with UV detection at 254 nm. The method was used for release testing of Calcium L-lactate to determine chiral purity and the content of Calcium D-lactate. The chemical structures of Calcium L-lactate and Calcium D-lactate are shown below.

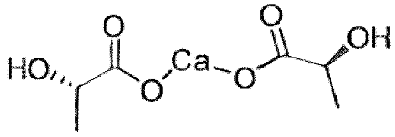

Calcium L-lactate

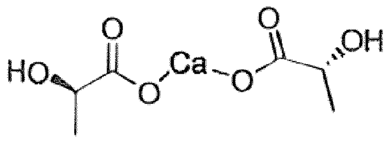

Calcium D-lactate

Column: Phenomenex Chirex 3126 (D)-penicillamine, 4.6mm ID x 250 mm L, 5 □m, Part no. 00G-3126-E0
Instrument: Agilent 1260 HPLC system with UV detector.
Mobile phase: 2 mmol/L $CuSO_4$ in water:IPA (98:2)
Column temp: 30 °C.
Flow rate: 0.7 mL/min
Injection volume: 10 μL
Detector: 254 nm
Needle wash solvent: diluent
Run time: 40 min

The relative retention times (RRT) for D- and L-lactic acid using this method were as follows.

| Peak | Reference Retention Time (min) | RRT |
|---|---|---|
| L-lactic acid | 24.493 | 1.00 |
| D-lactic acid | 32.190 | 1.31 |

Integration and calculations were carried out by only integrating the peaks for L-lactic acid and D-lactic acid in the chromatograms.

$$\%\ \text{Calcium D-lactate} = [A_{\text{D-lactic acid}} / (A_{\text{L-lactic acid}} + A_{\text{D-lactic acid}})] \times 100$$

$$\text{Chiral purity } (\%) = 100\ (\%) - \%\ \text{Calcium D-lactate}^{*}$$

* Only when D-lactic acid peak is ≥ 0.45% is it considered in the calculation.

FIG. 5 shows a representative chromatogram of a standard solution.

$^1$HNMR data (including QNMR data) was obtained on a Varian Mercury 400 mHz machine.

**Reaction Scheme 1:** Use of Concentrated $H_2SO_4$ as Mineral Acid

**Example 1**

In an exemplary embodiment of Reaction Scheme 1, concentrated $H_2SO_4$ (1.14 mL, 20.61 mmol, 0.9 eq.) was added drop wise to a cold (-14 °C) slurry of Ca-lactate hydrate (5 g, 22.9 mmol anhydrous basis, 1.0 eq.) and MeOAc (100 mL) in a flask under nitrogen. The slurry was stirred for 30 minutes at a temperature of -12 °C and for an additional 2 hours at room temperature. The resulting free flowing slurry was then filtered, and the filtrate was concentrated to produce 3.79 g (quantitative) of a viscous liquid. The filtrate of Example 1 can be further processed by isolating, recovering, filtering, concentrating, decanting, distilling, crystallizing and/or dehydrating the filtrate to produce anhydrous lactic acid.

**Example 2:**

In an exemplary embodiment of Reaction Scheme 1, concentrated $H_2SO_4$ (1.27 mmol, 22.9 mmol, 1.0 eq.) was added drop wise to a cold (-14 °C) slurry of Ca-lactate hydrate (10 g, 34.45 mmol $4H_2O$ basis, 1.5 eq.) and MeOAc (100 mL) in a flask under nitrogen. The reaction mixture was stirred for 3 h at a temperature of -12 °C to -14 °C and for an additional 2

hours at room temperature. The resulting slurry was then filtered through a sintered funnel and concentrated on a rotatory evaporator to approximately 3 volumes. 5 volumes of heptane were then added slowly, which formed an oily immiscible bottom layer in the reaction mixture. The reaction mixture was then concentrated to 3 volumes. 5 volumes of heptane were added resulting in a similar bottom oily layer. The reaction mixture was then completely concentrated to a viscous oil, dissolved in 50 mL MeOAc and dried over anhydrous $Na_2SO_4$. The mixture was then concentrated to 2 volumes. 2 volumes of heptane were added followed by lactic acid seed crystals. Commercially purchased anhydrous lactic acid was used as seed in the initial experiment, and the material of Example 2 or commercially purchased anhydrous lactic acid was used as seed in later experiments. The reaction mixture was then cooled in an ice bath while stirring, resulting in a semisolid sludge with some white crystals. The semisolid sludge was then completely concentrated and subjected to high vacuum filtration to produce 3.57 grams of hygroscopic white solid anhydrous lactic acid. This synthesis yielded 86.5% by weight anhydrous lactic acid.

**Example 3:**

In an exemplary embodiment of Reaction Scheme 1, concentrated $H_2SO_4$ (3.06 mL, 55.12 mmol, 1.0 eq.) was added drop wise to a cold (-13 °C) slurry of Ca-lactate hydrate (20 g, 68.9 mmol based on $4H_2O$ basis, 1.25 eq.) and MeOAc (200 mL) in a flask under nitrogen. The slurry was stirred for 3h at a temperature of -10°C to -14°C and for an additional 2 hours at room temperature. The free-flowing slurry was then filtered via a short pad of anhydrous $Na_2SO_4$ in a sintered funnel and concentrated on a rotary evaporator to approximately 2 volumes. The solution was then cooled to 0 °C followed by slowly adding 1.5 volume of heptane while stirring. At this point, L-lactic acid seed crystals and an additional 4.5 volumes of heptane (total 6 volume) were added. The resulting bilayer was then completely concentrated to a semisolid sludge. The semisolid was dissolved in 2 volumes of MeOAc, and 10 volumes of heptane were added slowly. An immiscible layer was formed at the bottom of the mixture. The mixture was then concentrated down to 2 volumes, and 10 volumes of heptane were added followed by additional seed crystals of lactic acid. A clumpy slurry was formed, which was completely concentrated to obtain white hygroscopic solid crystals of anhydrous L-lactic acid. The synthesis yielded 9.47 grams of 95.4% by weight anhydrous L-lactic acid.

**Example 4:**

In an exemplary embodiment of Reaction Scheme 1, concentrated $H_2SO_4$ (275.6 mmol, 1.0 eq.) was added drop wise over 15 minutes to a cold (-13 °C) slurry of Ca-lactate hydrate (100 g, 344.56 mmol based on $4H_2O$ basis, 1.25 eq.) and MeOAc (800 mL) in a flask under nitrogen. The slurry was stirred for 6h at a temperature of -12 °C to -14 °C. The slurry was then filtered via a Buchner funnel equipped with filter paper and cloth. The filter cake was washed with MeOAc (50 mL x2) and the total volume of 1000 mL of filtrate was produced. The filtrate was then divided into following fractions by measuring in a measuring cylinder:

1. Fraction A: 300 mL of the filtrate was used in crystallization from heptane/MeOAc.
2. Fraction B: 300 mL of the filtrate was used in crystallization from toluene/MeOAc.
3. Fraction C: 300 mL of the filtrate was used for crystallization from the aqueous solution.
4. Fraction D: 95mL was unused.

**Fraction A:**

The filtrate in aqueous solution of Fraction A was concentrated down to 30 mL, cooled with an ice bath to a temperature of 0 to 5 °C, and 60 mL heptane was added drop wise to form an oily immiscible layer. At this point, 30 mg of anhydrous lactic acid crystals were added which immediately dissolved. The whole solution was then concentrated down to 30 mL. 60 mL of heptane was added and concentrated down to 30 mL and repeated 3 times. 60 mL of heptane was then added, cooled in an ice bath followed by the addition of 30 mg anhydrous lactic acid as seed crystals to form a slurry. The slurry was not free flowing or filterable. The slurry was again completely concentrated in a rotary evaporator, dissolved in 10 mL MeOAc, cooled in an ice bath followed by the addition of seed crystals and 60 mL of heptane to produce a slurry with better properties for filtering. The slurry was then completely concentrated to get white solid of anhydrous L-lactic acid sticking on the wall. The synthesis resulted in 12.2 grams of 82% by weight of anhydrous L-lactic acid.

**Fraction B:**

The filtrate in aqueous solution of Fraction B was concentrated down to 30 mL, cooled in an ice bath to a temperature of 0 to 5 °C followed by a drop wise addition of 60 mL of toluene over 15 minutes. An oil immiscible layer was formed after the 60 mL of toluene was added. The bilayer was then concentrated down to 30 mL. To this solution, 60 mL

toluene was added and concentrated down to 30 mL at 30 °C and repeated 3 times. Finally, 60 mL of toluene was added, the solution was cooled in an ice bath to a temperature of 0 to 5 °C, followed by the addition of 30 mg anhydrous lactic acid seed crystals. The solution rapidly formed free-floating crystals with big chunks of anhydrous lactic acid. The solution was stirred until the big chunks of anhydrous lactic acid were broken and became a free-flowing filterable slurry. The slurry was then filtered under nitrogen to produce white crystals of anhydrous lactic acid. The synthesis formed 11.6 grams of 78% by weight anhydrous lactic acid.

**Fraction C:**

The filtrate in aqueous solution of Fraction C was completely concentrated to a viscous liquid, which was divided into two parts of 6 g each in 15 mL centrifuge tubes. 600 μL $H_2O$ was then added to both to make approximately 90% aqueous solutions. The solutions were then cooled to 15 °C, and anhydrous lactic acid seed crystals were added. The seeds dissolved at this temperature. The solutions were then gradually cooled to 10 °C and additional anhydrous lactic acid seed crystals were added, resulting in a slurry. The slurry was left in a fridge at a temperature of 6 °C, and the seeds grew to bigger crystals. The slurry was then centrifuged at a temperature of 5 °C and 3000 rpm for 15 minutes. The resulting solid with crystals was then collected by decantation and dried in a vacuum oven at room temperature. A total of 3.9 g (29% yield) of solid was collected. The crystals were hard to transfer and dry relative to Fractions B-C. The supernatant liquid present in the wet crystals was azeotroped with toluene (20 mL x 4). The resulting bilayer suspension with 20 mL toluene was cooled to a temperature of 0 °C followed by the addition of lactic acid seed crystals. A slurry was formed, which was distilled with 20 mL toluene two times. The resulting free flowing slurry was filtered under nitrogen to obtain 9.1 grams of 61% by weight anhydrous lactic acid in the form of white crystals.

**Example 5:**

In an exemplary embodiment of Reaction Scheme 1, concentrated $H_2SO_4$ (15 mL, 275.6 mmol, 1.0 eq.) was added drop wise for 30 minutes to a cold (-15 °C) slurry of Ca-lactate hydrate (100 g, 344.56 mmol based on $4H_2O$ basis, 1.25 eq.) and MeOAc (800 mL) in a flask under nitrogen. The slurry was stirred for 6 hours at a temperature of -12 °C to -14 °C. The slurry was then filtered via a Buchner funnel equipped with filter paper and cloth. The filter cake was washed with 100 mL MeOAc. The filtrate was concentrated to 300 mL. 200 mL of toluene was added, and the slurry was concentrated to 300 mL at a temperature of 30 °C. This step was repeated 3 times. The resulting bilayer was cooled in an ice bath to a temperature of 4 °C and centrifuged at 400 rpm as anhydrous lactic acid crystals (50 mg) were added as seed. A solid was immediately formed with chunks. The suspension was stirred at room temperature for 15 hours. A sticky slurry was formed that was not free-flowing or filterable. 2 volumes of MeOAc were added to dissolve all solids, and the solution was concentrated on a rotary evaporator until it became a viscous oil. 1 volume of MeOAc and 2 volumes of toluene were added to the solution and concentrated down to 2 volumes. 2 additional volumes of toluene were added and concentrated down to 3 volumes. A free-flowing slurry was formed at this point, which was filtered under vacuum and dried under high vacuum for 4 hours to produce 37.2 grams of 75% by weight anhydrous lactic acid in the form of a white crystalline solid. The water content of the anhydrous lactic acid product was 0.562% by weight as determined by Karl Fischer titration. FIG. 2 depicts the resulting anhydrous lactic acid product of the synthesis.

**Example 6:**

In an exemplary embodiment of Reaction Scheme 1, concentrated $H_2SO_4$ (15.0 mL, 275.6 mmol, 1.0 eq.) was added drop wise over 30 minutes to a cold (-13 °C) slurry of Ca-lactate hydrate (100 g, 344.56 mmol based on $4H_2O$ basis, 1.25 eq.) and MeOAc (800 mL) in a flask under nitrogen. The slurry was stirred for 6 hours at a temperature of -10°C to -12°C. The slurry was then filtered via a Buchner funnel equipped with filter paper and cloth. The filter cake was washed with 100 mL MeOAc. The filtrate was concentrated to 100 mL. 200 mL of MeOAc was added and concentrated down to 100 mL and this was repeated. 200 mL of toluene was added, concentrated to 300 mL at a temperature of 30 °C and repeated 3 times. 200 mL of toluene was added. The resulting bilayer was stirred at room temperature and anhydrous lactic acid crystals (50 mg) were added as seed crystals. A sludge-like slurry was formed that was not free-flowing or filterable. The slurry was concentrated to 100 mL at a temperature of 30 °C. 200 mL of toluene was added, concentrated down to 100 mL and this was repeated 2 times. A free-flowing slurry was formed at this point, which was filtered under vacuum and dried under high vacuum at room temperature for 24 hours to obtain 40.8 grams of 82% by weight anhydrous lactic acid in the form of a white crystalline solid.

Reaction Scheme 1 and the utilization of sulfuric acid produced high grade, high purity and high yields of anhydrous lactic acid from a slurry of Ca-lactate hydrate. The slurries produced by Reaction Scheme 1 were readily filtered and lactic acid was isolated in a purified end-product with high yields. Exemplary Reaction Scheme 1 can produce high purity and high yields of anhydrous lactic acid.

**Reaction Scheme 3:** Use of Solid Oxalic Acid as a Neutralizing Acid

**Example 7**

In an exemplary embodiment of Reaction Scheme 3, solid anhydrous oxalic acid (1.39 g, 15.5 mmol, 0.9 eq.) was added to a cold (-10 °C) slurry of calcium-L lactate hydrate (5 g, 1.0 equivalent, x mmol based on $4H_2O$) in MeOAc (100 mL). The resulting slurry was stirred for 40 minutes at a temperature of - 10 °C followed by stirring for 46 hours at room temperature. The slurry was then filtered through a sintered funnel and concentrated on a rotary evaporator to produce 2.63 g of 94% by weight of lactic acid in the form of a thick viscous liquid. The filtrate containing lactic acid can be further processed by isolating, recovering, filtering, concentrating, decanting distilling, crystallizing and/or dehydrating the filtrate to produce high purity anhydrous lactic acid.

**Example 8**

In an exemplary embodiment of Reaction Scheme 3, solid anhydrous oxalic acid (2.48 g, 27.54 mmol, 0.8 eq.) was added to a cold (-10 °C) slurry of calcium-L-lactate hydrate (10 g, 1.0 equivalent, 17.22 mmol based on $4H_2O$) in MeOAc (200 mL). The resulting slurry was warmed to room temperature and stirred for 18 hours at room temperature. The slurry was then filtered through a sintered funnel and concentrated on a rotary evaporator to 5 volumes to produce a filtrate. 5 volumes of heptane followed by lactic acid seed crystals were added to the filtrate. A sludge was formed, which was completely concentrated and kept in high vacuum to produce 3.86 g of 79% by weight anhydrous L-lactic acid in the form of a white crystalline solid.

Exemplary Reaction Scheme 3 can produce high yields of high purity anhydrous lactic acid, including L-(+)-lactic acid, D-(-)-lactic acid and/or DL-lactic acid.

The exemplary large-scale Synthesis Route (II) can be utilized to produce anhydrous L-lactic acid with less than 0.1% by weight water and less than 1% by weight of polylactate impurities, such as lactide, oligomers of lactic acid and mixtures thereof.

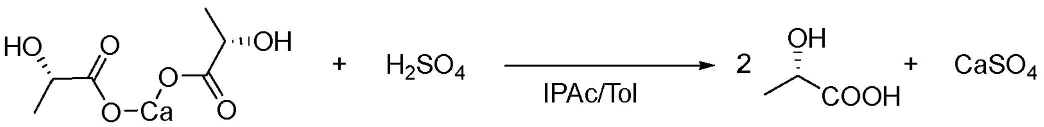


$C_6H_{10}CaO_6$ 218.22 Calcium L-lactate

$C_3H_6O_3$ 90.08

**Synthesis Route II**

FIG. 3 depicts an exemplary equipment layout for the synthesis of anhydrous lactic acid utilizing Synthesis Route II.

The equipment used in FIG. 3 includes two 80 L glass reactors (reactor 1 and reactor 2) and a tray dryer. The reactors are equipped with stirring apparatus. A filter flask is used for filtration. Vacuum filtration or pressure filtration can also be employed.

Typical reactants, solvents and materials for conducting Synthesis Route II are outlined in Table 1.

**Table 1: Material Dispensing Summary for Synthesis Route (II)**

| Material | Mol. Wt. | Quantity (kg) | Mol. | Molar Ratio | Weight Ratio | Spec |
|---|---|---|---|---|---|---|
| Calcium L-lactate | 218.2 | 7.5 | 34.4 | 1.0 | 1.0 | white solid; conform to structure QNMR Assay: ≥98% Chiral HPLC purity:≥98% |
| Isopropyl acetate | - | 55.5 | - | - | 7.4 | - |
| Concentrated sulfuric acid | 98.1 | 2.8 | 28.5 | 0.8 | 0.4 | - |
| Toluene | - | 65.1 | - | - | 8.7 | - |

(continued)

| Material | Mol. Wt. | Quantity (kg) | Mol. | Molar Ratio | Weight Ratio | Spec |
|---|---|---|---|---|---|---|
| Seed crystal | 98.1 | 0.03 | 0.3 | 0.0 | 0.0 | - |

In Synthesis Route II, before charging reactor 1 with the reactants, the oxygen content in the reactor is reduced to ≤ to 1% (by volume). In an exemplary embodiment, the oxygen content in reactor is 0.7% prior to initiating the reaction.

Reactor 1 is charged with 34.7 kg of isopropyl acetate and the stirring apparatus is started. The reactor was then charged with 7.5 kg anhydrous calcium L-lactate. The reaction mixture is maintained at a temperature of 0-10 °C. In an exemplary embodiment, the reaction mixture is cooled and maintained at 7.3 °C.

The reactor is then charged with 2.8 kg of sulfuric acid at a temperature of 0-10 °C. The reaction temperature is maintained at 0-10 °C during the reaction. After 3 hours, a sample of the mixture is analyzed every 1-4 hours using $^1$H-NMR spectroscopy until assay by QNMR[1] is greater than or equal to 11.5 or the difference between two consecutive samples is ≤ 0.5%.

In an exemplary embodiment the reaction is carried out for 7 h 10 min, the assay by QNMR[1] yielded 8.8%, the assay by QNMR[2] yielded 8.5% and the difference between two consecutive samples was 0.3%. The peak area of the lactic acid peak at 4.07 ppm was monitored for the quantitative NMR analysis. QNMR stands for quantitative $^1$HNMR where the amount of the test material was quantified (assayed) against an internal reference standard. In this case the internal reference standard was 1,3,5-trimethoxybenzene.

The reaction mixture is then filtered in a first filtration with a filter flask and a filter cake is generated. Vacuum filtration can be employed.

Reactor 1 is charged a second time with 13.9 kg of isopropyl acetate at a temperature of 0-30 °C. In an exemplary embodiment, reactor 1 is charged a second time with isopropyl acetate at a temperature between 3.7-6.4 °C. The isopropyl acetate is cooled to a temperature between 0-10 °C. In an exemplary embodiment, the mixture is cooled to a temperature of between 6.3 °C.

The filter cake from the first filtration was added to reactor 1 at a temperature of 1-10 °C. In an exemplary embodiment, the filter cake was added to reactor 1 at a temperature of 6.3-6.4 °C. The reaction mixture is stirred for 1-2 h at a temperature of 0-10 °C. In an exemplary embodiment, the reaction mixture is stirred for 1h 3min at a temperature of 6.4-8.7 °C.

The reaction mixture is filtered in a second filtration with a filter flask. The filtrate from the first filtration and the filtrate from the second filtration are added to reactor 2 through capsule filter in portions.

The mixture in reactor 2 is concentrated to ≤ to 45 °C under reduced pressure (P≤-0.08 MPa) until 0.5-1.0 volume is left. In an exemplary embodiment, the mixture is concentrated at a temperature of 16.5-32 °C and a pressure of -0.08 MPa.

Reactor 2 is additionally charged with 6.9 kg of isopropyl acetate and was stirred until fully dissolved. The mixture was sampled using Karl Fisher titration to determine water content. The mixture is cooled to a temperature of 20-25 °C. In an exemplary embodiment, the mixture is cooled to a temperature of 24.3 °C.

Anhydrous L-lactic acid seed crystals are added to the mixture. In an exemplary embodiment, 30.0g of anhydrous L-lactic acid seed crystals are added to the mixture at a temperature of 20-25 °C for 1-2h. The mixture is stirred at a temperature of 20-25 °C for 1-2 h. In an exemplary embodiment, the mixture is stirred at a temperature of 21.4-24.2 °C for 1-2 h.

The mixture is cooled to a temperature of 0-10 °C. In an exemplary embodiment, the mixture is cooled to a temperature of 9.5 °C. The mixture is stirred at a temperature of 0-10 °C for 1-2 h. In an exemplary embodiment, the mixture is stirred at a temperature of 2.3-9.5 °C.

At a temperature of 0-10 °C, toluene is added to the mixture through capsule filter. In an exemplary embodiment 52.2 kg of toluene is added to the mixture through capsule filter at a temperature of 0-10°C.

The mixture is stirred at a temperature of 0-10 °C for 2-3 h. In an exemplary embodiment, the mixture is stirred at a temperature of 1-2.8 °C for 2-3 h.

The mixture was filtered with a filter flask. The filter cake was rinsed with 6.3 kg of toluene twice. The solid from the filter is placed in a tray dryer and dried at 15-25 °C for 12 h. In an exemplary embodiment, the drying temperature is 20-21 °C. The solid is sampled for residual solvent analysis every 4-12 h until residual isopropyl acetate is ≤ 5000 ppm and the residual toluene is ≤ 890 ppm. In an exemplary embodiment, the product contained 563 ppm isopropyl acetate and 277 ppm toluene.

The resulting product is an anhydrous L-lactic acid. In an exemplary embodiment, the resulting product is 6.0 kg of 99.3% by weight anhydrous L-lactic acid (based on $^1$H-NMR spectroscopy). The yield of Synthesis Route II is at least 47% and has a chiral purity of 100%.

Reaction scale and yield for two batches of anhydrous L-lactic acid produced by Synthesis Route II are provided

in Table 2.

**Table 2: Synthesis Route II Results**

| Batch No. | Scale (Reactor Size, L) | Ca-L-lactate (kg) | Anhydrous Lactic Acid (kg) | | Assay by $^1$HNMR | Yield (%) |
|---|---|---|---|---|---|---|
| | | | Theoretical | Actual | | |
| | 80 | 7.5 | 12.4 | 6.0 | 99.3% | 48.3% |
| **Batch # 1** | | 7.5 | | | | |
| **Batch # 2** | 80 | 7.5<br>7.5 | 12.4 | 5.9 | 100% | 47.6% |

Tables 3 and 4 summarize the chemical properties of the anhydrous lactic acid end products of Batches 1 and 2.

**Table 3: Chemical Properties of Anhydrous Lactic Acid End Product**

| Batch | Yield | appearance | Assay ($^1$HNMR) | Assay (NaOH titr) | KF | Specific optical Rot. | ROI | Ca residue |
|---|---|---|---|---|---|---|---|---|
| **Batch # 1** | 6.04 Kg | white | 99 % | 99.4% | <0.04% | -14.2° | 0.04% | 0.0% |
| **Batch # 2** | 5.89 Kg | white | 99.2 % | 99.7% | <0.06% | -14.1° | 0.01% | 0% |

**Table 4: Chemical Properties of Anhydrous Lactic Acid End Product**

| Batch | Yield | AcOH | iPrOAc | Toluene | Benzene | Sulfur content | MP | Chiral HPLC |
|---|---|---|---|---|---|---|---|---|
| **Batch # 1** | 6.04 Kg | <LOD (ND) | ND (LOD= 167ppm) | ND (LOD= 167ppm) | < 2 ppm | 0.01 % | 52°C | 99.99% |
| **Batch # 2** | 5.89 Kg | 1418 | < 500 | < 500 | < 2 ppm | 0% | 54°C | 99.98% |

FIG. 4 depicts $^1$HNMR results for an L-(+)-Lactic Acid end-product synthesized using Synthesis Route II.

The exemplary synthesis and reaction schemes disclosed herein can be used to produce anhydrous lactic acid, and specifically L-(+)-lactic acid, D-(-)-lactic acid and DL-Lactic acid for use in industrial processes, including but not limited to petrochemical processes; chemical and polymer synthesis; processes for the production of cosmetics and personal care products; processes for the production of food additives, decontaminants and flavoring agents; and processes for the production of pharmaceuticals and active pharmaceutical ingredients.

## Claims

**1.** A method of synthesizing anhydrous L-(+)-lactic acid, D-(-)-lactic acid, or DL-lactic acid comprising:

reacting a compound of formula (Ia), or an enantiomer thereof:

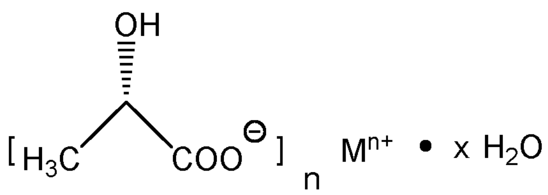

(Ia)

with an acid compound of formula $H_nX$ in a first solvent to form a reaction mixture comprising a compound of formula (Ib) and a lactic acid compound of formula (I), or an enantiomer thereof, in solution with the first solvent:

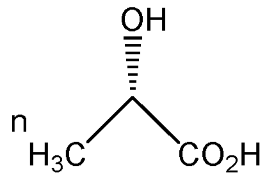

(I)

$MX_n \bullet x\ H_2O$

(Ib)

wherein each n is independently an integer other than 0, x is 0, or an integer other than 0,
M is an alkali metal or alkaline earth metal and X is the conjugate base of the acid compound of formula $H_nX$; and

filtering the reaction mixture to produce a filtrate containing the lactic acid compound of formula (I), or an enantiomer thereof, in solution.

2. The method of claim 1, further comprising concentrating the filtrate, optionally wherein concentrating the filtrate preferably comprises distilling the filtrate.

3. The method of claim 2, further comprising crystallizing the filtrate from a second solvent to produce a crystallized product, wherein the second solvent is preferably toluene or heptane.

4. The method of claim 1, wherein the lactic acid compound of formula (I), or an enantiomer thereof, is soluble in the first solvent and/or the compound of formula (Ib) is insoluble in the first solvent.

5. The method of claim 1, wherein M is selected from the group consisting of calcium, sodium, potassium, magnesium, lithium, barium, beryllium, cesium and strontium.

6. The method of claim 1, wherein

(i) the acid compound of formula $H_nX$ is sulfuric acid and X is sulfate;
(ii) the acid compound of formula $H_nX$ is oxalic acid and X is oxalate; or
(iii) the acid compound of formula $H_nX$ is hydrochloric acid and X is chloride.

7. The method of claim 1, wherein the compound of formula (Ia), or an enantiomer thereof, is calcium-L-lactate hydrate.

8. The method of claim 1, wherein the first solvent is selected from the group consisting of acetone, toluene, acetonitrile, MTBE, isopropanol, ethanol, methanol and tetrahydrofuran, or wherein the first solvent is an ester solvent, which is preferably methyl acetate, ethyl acetate, isopropyl acetate, or n-propyl acetate.

9. The method of claim 3, further comprising introducing seed crystals of anhydrous L-(+)-lactic acid, D-(-)-lactic acid, or DL-lactic acid to the second solvent during crystallization.

10. The method of claim 1, further comprising azeotropically removing water from the filtrate.

11. The method of claim 3, further comprising vacuum drying the crystallized product.

12. The method of claim 1, further comprising drying the filtrate with a drying agent, which is preferably $Na_2SO_4$.

13. A method of synthesizing anhydrous L-(+)-lactic acid, D-(-)-lactic acid, or DL-lactic acid comprising reacting anhydrous calcium lactate with an acid in an ester solvent.

14. The method of claim 13, wherein the acid is sulfuric acid, wherein the ester solvent is isopropyl acetate.

15. The method of claim 3 or 13, wherein the anhydrous L-(+)-lactic acid, D-(-)-lactic acid, or DL-lactic acid comprises less

than 1% by weight polylactate impurities.

**16.** The method of claim 13, wherein the anhydrous L-(+)-lactic acid, D-(-)-lactic acid, or DL-lactic acid comprises less than 0.1% by weight water.

**17.** A method for the preparation of a compound of Formula (XXIII):

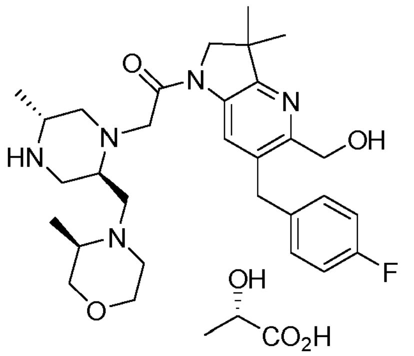

(XXIII)

the method comprising

reacting a compound of formula (Ia):

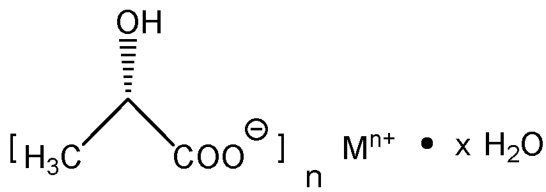

(Ia)

with an acid compound of formula $H_nX$ in a first solvent to form a reaction mixture comprising a compound of formula (Ib) and a lactic acid compound of formula (I) in solution with the first solvent:

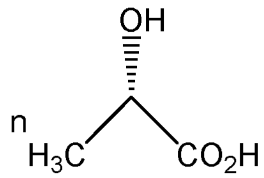

$MX_n \bullet x\ H_2O$

(I) (Ib)

wherein each n is independently an integer other than 0, x is 0, or an integer other than 0,
M is an alkali metal or alkaline earth metal and X is the conjugate base of the acid compound of formula $H_nX$;

filtering the reaction mixture to produce a filtrate containing the lactic acid compound of formula (I) in solution; and contacting a compound of Formula (XXII)

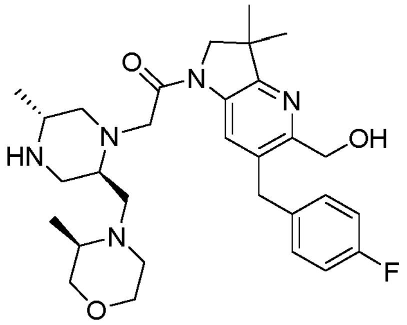

(XXII)

with the anhydrous lactic acid compound of formula (I) to provide the compound of Formula (XXIII).

## Patentansprüche

1. Verfahren zur Synthese von wasserfreier L-(+)-Milchsäure, D-(-)-Milchsäure oder DL-Milchsäure, umfassend:

   das Umsetzen einer Verbindung mit der Formel (Ia) oder eines Enantiomers davon:

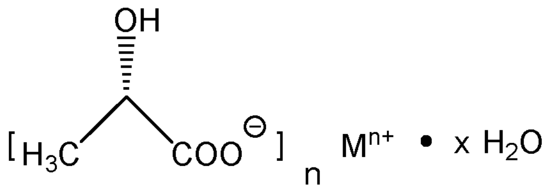

(Ia)

   mit einer Säureverbindung mit der Formel $H_nX$ in einem ersten Lösungsmittel, um ein Reaktionsgemisch zu bilden, das eine Verbindung mit der Formel (Ib) und eine Milchsäureverbindung mit der Formel (I) oder ein Enantiomer davon in Lösung mit dem ersten Lösungsmittel umfasst:

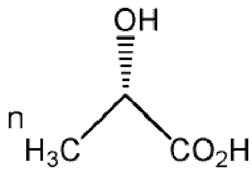

(I)

$MX_n \bullet x\ H_2O$

(Ib)

   wobei jedes n unabhängig eine ganze Zahl ungleich 0 ist, x 0 oder eine ganze Zahl ungleich 0 ist, M ein Alkalimetall oder Erdalkalimetall ist und X die Konjugatbase der Säureverbindung mit der Formel $H_nX$ ist; und das Filtrieren des Reaktionsgemisches, um ein Filtrat herzustellen, das die Milchsäureverbindung mit der Formel (I) oder ein Enantiomer davon in Lösung enthält.

2. Verfahren nach Anspruch 1, ferner umfassend das Konzentrieren des Filtrats, optional, wobei das Konzentrieren des Filtrats vorzugsweise das Destillieren des Filtrats umfasst.

3. Verfahren nach Anspruch 2, ferner umfassend das Kristallisieren des Filtrats aus einem zweiten Lösungsmittel, um ein kristallisiertes Produkt herzustellen, wobei das zweite Lösungsmittel vorzugsweise Toluol oder Heptan ist.

4. Verfahren nach Anspruch 1, wobei die Milchsäureverbindung mit der Formel (I) oder ein Enantiomer davon in dem ersten Lösungsmittel löslich ist und/oder die Verbindung mit der Formel (Ib) in dem ersten Lösungsmittel unlöslich ist.

5. Verfahren nach Anspruch 1, wobei M aus der Gruppe, bestehend aus Calcium, Natrium, Kalium, Magnesium, Lithium, Barium, Beryllium, Cäsium und Strontium, ausgewählt ist.

6. Verfahren nach Anspruch 1, wobei

(i) die Säureverbindung mit der Formel $H_nX$ Schwefelsäure ist und X Sulfat ist;
(ii) die Säureverbindung mit der Formel $H_nX$ Oxalsäure ist und X Oxalat ist; oder
(iii) die Säureverbindung mit der Formel $H_nX$ Chlorwasserstoffsäure ist und X Chlorid ist.

7. Verfahren nach Anspruch 1, wobei die Verbindung mit der Formel (Ia) oder ein Enantiomer davon Calcium-L-lactathydrat ist.

8. Verfahren nach Anspruch 1, wobei das erste Lösungsmittel aus der Gruppe, bestehend, aus Aceton, Toluol, Acetonitril, MTBE, Isopropanol, Ethanol, Methanol und Tetrahydrofuran, ausgewählt ist oder wobei das erste Lösungsmittel ein Esterlösungsmittel ist, das vorzugsweise Methylacetat, Ethylacetat, Isopropylacetat oder n-Propylacetat ist.

9. Verfahren nach Anspruch 3, ferner umfassend das Einbringen der Impfkristalle von wasserfreier L-(+)-Milchsäure, D-(-)-Milchsäure oder DL-Milchsäure in das zweite Lösungsmittel während der Kristallisation.

10. Verfahren nach Anspruch 1, ferner umfassend das azeotrope Entfernen von Wasser aus dem Filtrat.

11. Verfahren nach Anspruch 3, ferner umfassend das Vakuumtrocknen des kristallisierten Produkts.

12. Verfahren nach Anspruch 1, ferner umfassend das Trocknen des Filtrats mit einem Trocknungsmittel, das vorzugsweise $Na_2SO_4$ ist.

13. Verfahren zur Synthese von wasserfreier L-(+)-Milchsäure, D-(-)-Milchsäure oder DL-Milchsäure, umfassend das Umsetzen von wasserfreiem Calciumlactat mit einer Säure in einem Esterlösungsmittel.

14. Verfahren nach Anspruch 13, wobei die Säure Schwefelsäure ist, wobei das Esterlösungsmittel Isopropylacetat ist.

15. Verfahren nach Anspruch 3 oder 13, wobei die wasserfreie L-(+)-Milchsäure, D-(-)-Milchsäure oder DL-Milchsäure weniger als 1 Gew.-% Polylactatverunreinigungen umfasst.

16. Verfahren nach Anspruch 13, wobei die wasserfreie L-(+)-Milchsäure, D-(-)-Milchsäure oder DL-Milchsäure weniger als 0,1 Gew.-% Wasser umfasst.

17. Verfahren zur Herstellung einer Verbindung mit der Formel (XXIII):

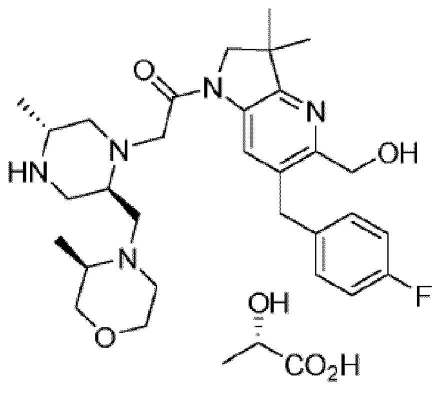

(XXIII)

wobei das Verfahren umfasst
das Umsetzen einer Verbindung mit der Formel (Ia):

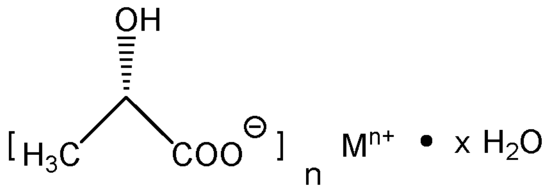

(Ia)

mit einer Säureverbindung mit der Formel $H_nX$ in einem ersten Lösungsmittel, um ein Reaktionsgemisch zu bilden, das eine Verbindung mit der Formel (Ib) und eine Milchsäureverbindung mit der Formel (I) in Lösung mit dem ersten Lösungsmittel umfasst:

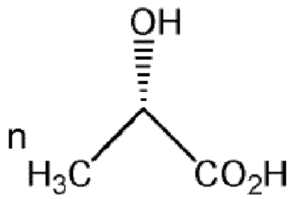

$MX_n \bullet x\ H_2O$

(I) (Ib)

wobei jedes n unabhängig eine ganze Zahl ungleich 0 ist, x 0 oder eine ganze Zahl ungleich 0 ist, M ein Alkalimetall oder Erdalkalimetall ist und X die Konjugatbase der Säureverbindung mit der Formel $H_nX$ ist;
das Filtrieren des Reaktionsgemisches, um ein Filtrat zu erhalten, das die Milchsäureverbindung mit der Formel (I) in Lösung enthält; und
das Inkontaktbringen einer Verbindung mit der Formel (XXII)

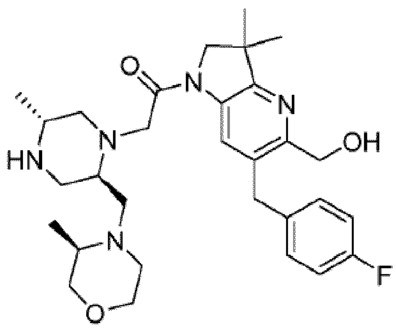

(XXII)

mit der wasserfreien Milchsäureverbindung mit der Formel (I), um die Verbindung mit der Formel (XXIII) bereitzustellen.

## Revendications

**1.** Procédé de synthèse d'acide L-(+)-lactique, d'acide D-(-)-lactique ou d'acide DL-lactique anhydre comprenant:

la mise en réaction d'un composé de formule (Ia) ou d'un énantiomère de celui-ci:

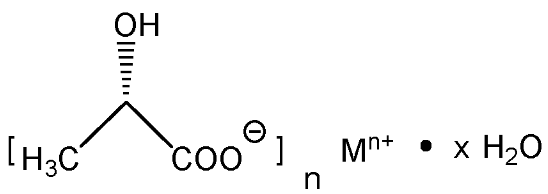

(Ia)

avec un composé d'acide de formule $H_nX$ dans un premier solvant pour former un mélange réactionnel comprenant un composé de formule (Ib) et un composé d'acide lactique de formule (I), ou un énantiomère de celui-ci, en solution avec le premier solvant:

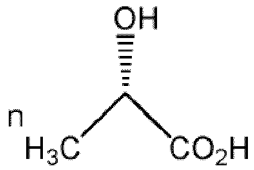

$MX_n \bullet x\ H_2O$

(I) (Ib)

dans lequel chaque n est indépendamment un nombre entier autre que 0, x est 0 ou un nombre entier autre que 0, M est un métal alcalin ou un métal alcalino-terreux et X est la base conjuguée du composé d'acide de formule $H_nX$; et

la filtration du mélange réactionnel pour produire un filtrat contenant le composé d'acide lactique de formule (I), ou un énantiomère de celui-ci, en solution.

**2.** Procédé selon la revendication 1, comprenant en outre la concentration du filtrat, optionnellement où la concentration du filtrat comprend de préférence la distillation du filtrat.

**3.** Procédé selon la revendication 2, comprenant en outre la cristallisation du filtrat à partir d'un deuxième solvant pour donner un produit cristallisé, dans lequel le deuxième solvant est de préférence le toluène ou l'heptane.

**4.** Procédé selon la revendication 1, dans lequel le composé d'acide lactique de formule (I), ou un énantiomère de celui-ci, est soluble dans le premier solvant et/ou le composé de formule (Ib) est insoluble dans le premier solvant.

**5.** Procédé selon la revendication 1, dans lequel M est choisi dans le groupe constitué de: calcium, sodium, potassium, magnésium, lithium, baryum, béryllium, césium et strontium.

**6.** Procédé selon la revendication 1, dans lequel

(i) le composé d'acide de formule $H_nX$ est l'acide sulfurique et X est un sulfate;
(ii) le composé d'acide de formule $H_nX$ est l'acide oxalique et X est un oxalate; ou
(iii) le composé d'acide de formule $H_nX$ est l'acide chlorhydrique et X est un chlorure.

**7.** Procédé selon la revendication 1, dans lequel le composé de formule (Ia), ou un énantiomère de celui-ci, est de l'hydrate de calcium-L-lactate.

**8.** Procédé selon la revendication 1, dans lequel le premier solvant est choisi dans le groupe constitué de : acétone, toluène, acétonitrile, MTBE, isopropanol, éthanol, méthanol et tétrahydrofurane ou dans lequel le premier solvant est un solvant ester, qui est de préférence l'acétate de méthyle, l'acétate d'éthyle, l'acétate d'isopropyle ou l'acétate de n-propyle.

**9.** Procédé selon la revendication 3, comprenant en outre l'introduction de cristaux germes d'acide L-(+)-lactique, d'acide D-(-)-lactique ou d'acide DL-lactique anhydre au deuxième solvant pendant la cristallisation.

**10.** Procédé selon la revendication 1, comprenant en outre le retrait azéotropique d'eau à partir du filtrat.

**11.** Procédé selon la revendication 3, comprenant en outre le séchage sous vide du produit cristallisé.

**12.** Procédé selon la revendication 1, comprenant en outre le séchage du filtrat avec un agent séchant, qui est de préférence $Na_2SO_4$.

**13.** Procédé de synthèse d'acide L-(+)-lactique, d'acide D-(-)-lactique ou d'acide DL-lactique anhydre comprenant la mise en réaction de lactate de calcium anhydre avec un acide dans un solvant ester.

**14.** Procédé selon la revendication 13, dans lequel l'acide est l'acide sulfurique, dans lequel le solvant ester est l'acétate d'isopropyle.

**15.** Procédé selon la revendication 3 ou 13, dans lequel l'acide L-(+)-lactique, l'acide D-(-)-lactique ou l'acide DL-lactique anhydre comprend moins de 1% en poids d'impuretés de polylactate.

**16.** Procédé selon la revendication 13, où l'acide L-(+)-lactique, l'acide D-(-)-lactique ou l'acide DL-lactique anhydre comprend moins de 0,1% en poids d'eau.

**17.** Procédé de préparation d'un composé de Formule (XXIII):

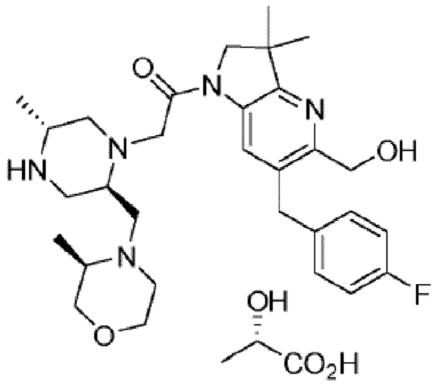

(XXIII)

le procédé comprenant:

la mise en réaction d'un composé de formule (Ia):

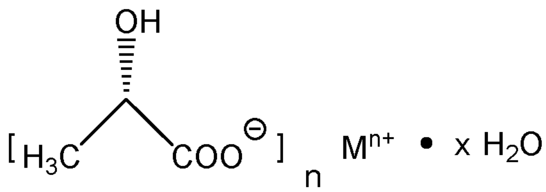

(Ia)

avec un composé d'acide de formule $H_nX$ dans un premier solvant pour former un mélange réactionnel comprenant un composé de formule (Ib) et un composé d'acide lactique de formule (I) en solution avec le premier solvant:

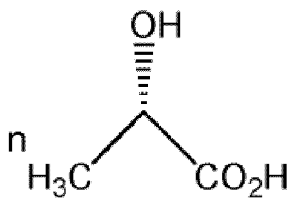

$MX_n \bullet x\ H_2O$

(I) (Ib)

dans lequel chaque n est indépendamment un nombre entier autre que 0, x est 0 ou un nombre entier autre que 0, M est un métal alcalin ou un métal alcalino-terreux et X est la base conjuguée du composé d'acide de formule $H_nX$;
la filtration du mélange réactionnel pour produire un filtrat contenant le composé d'acide lactique de formule (I) en solution; et
la mise en contact d'un composé de Formule (XXII)

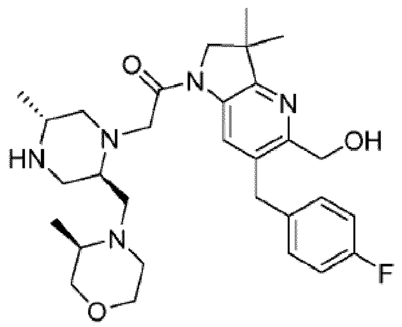

(XXII)

avec le composé d'acide lactique anhydre de formule (I) pour donner le composé de Formule (XXIII).

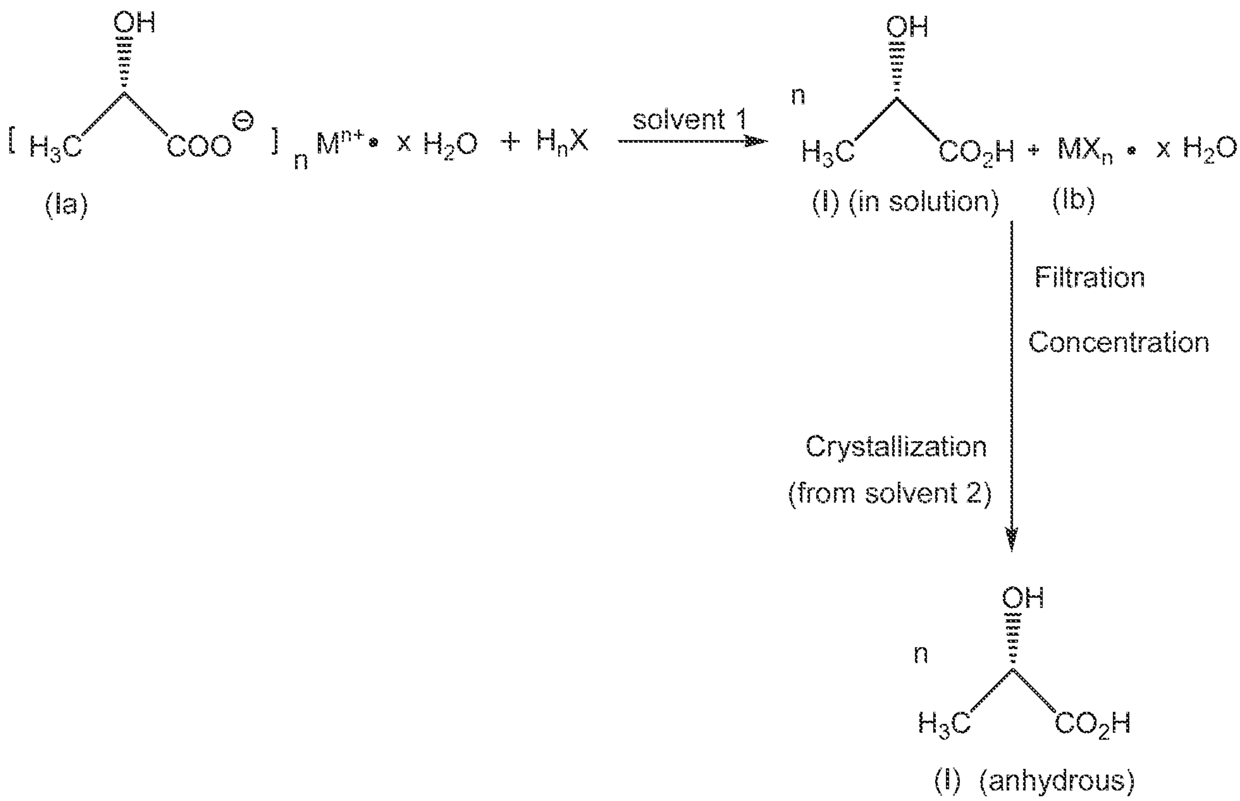
solvent 1
Filtration
Concentration
Crystallization
(from solvent 2)

FIG. 1

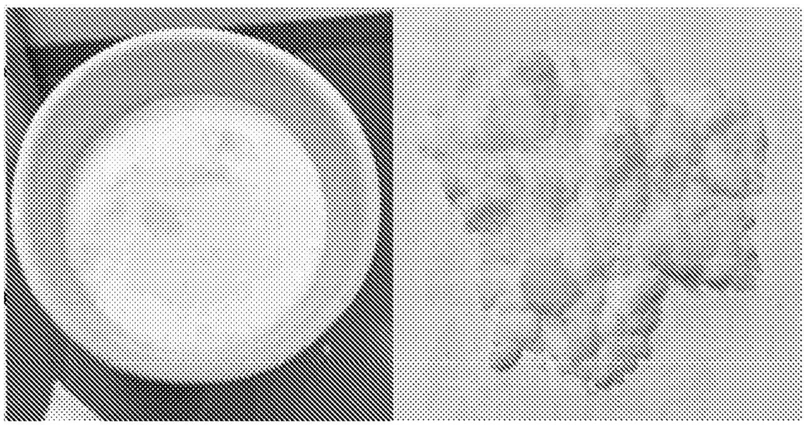

FIG. 2

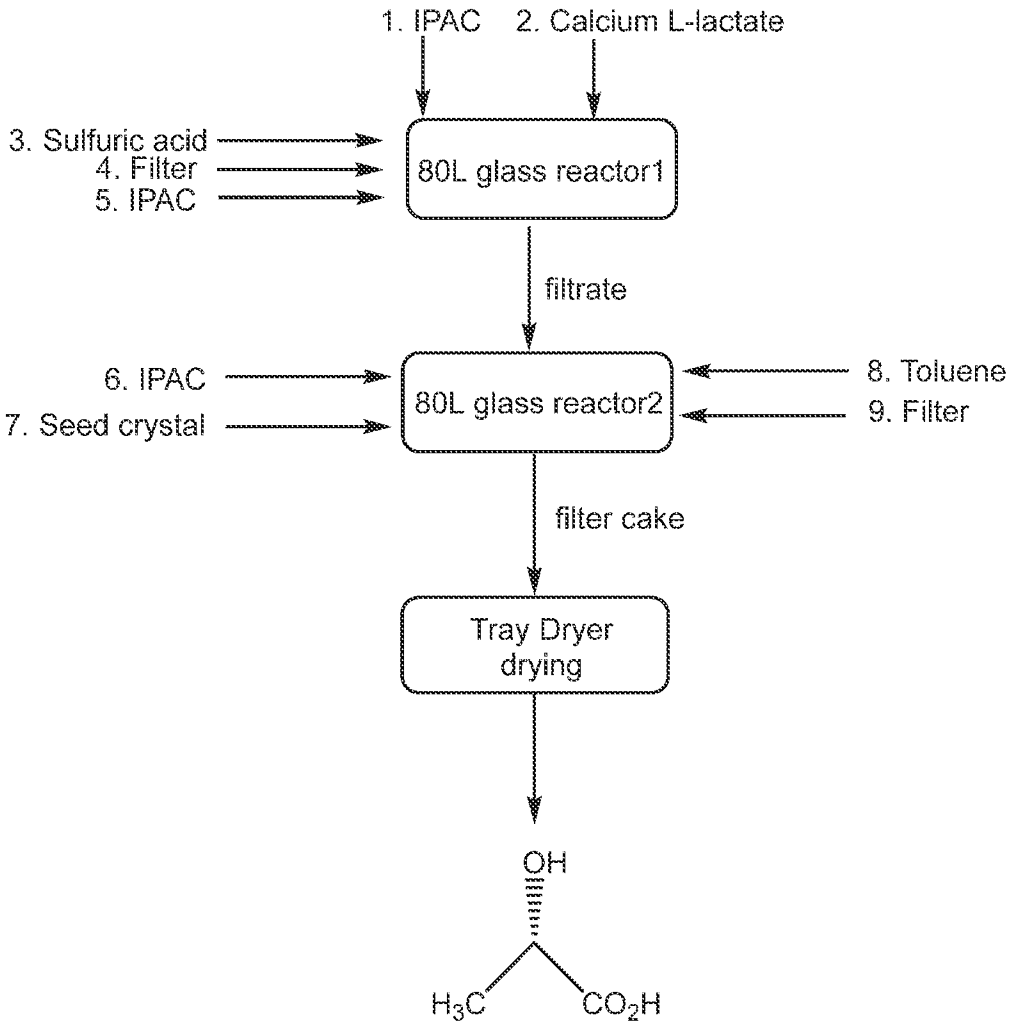


(I) (anhydrous)

FIG. 3

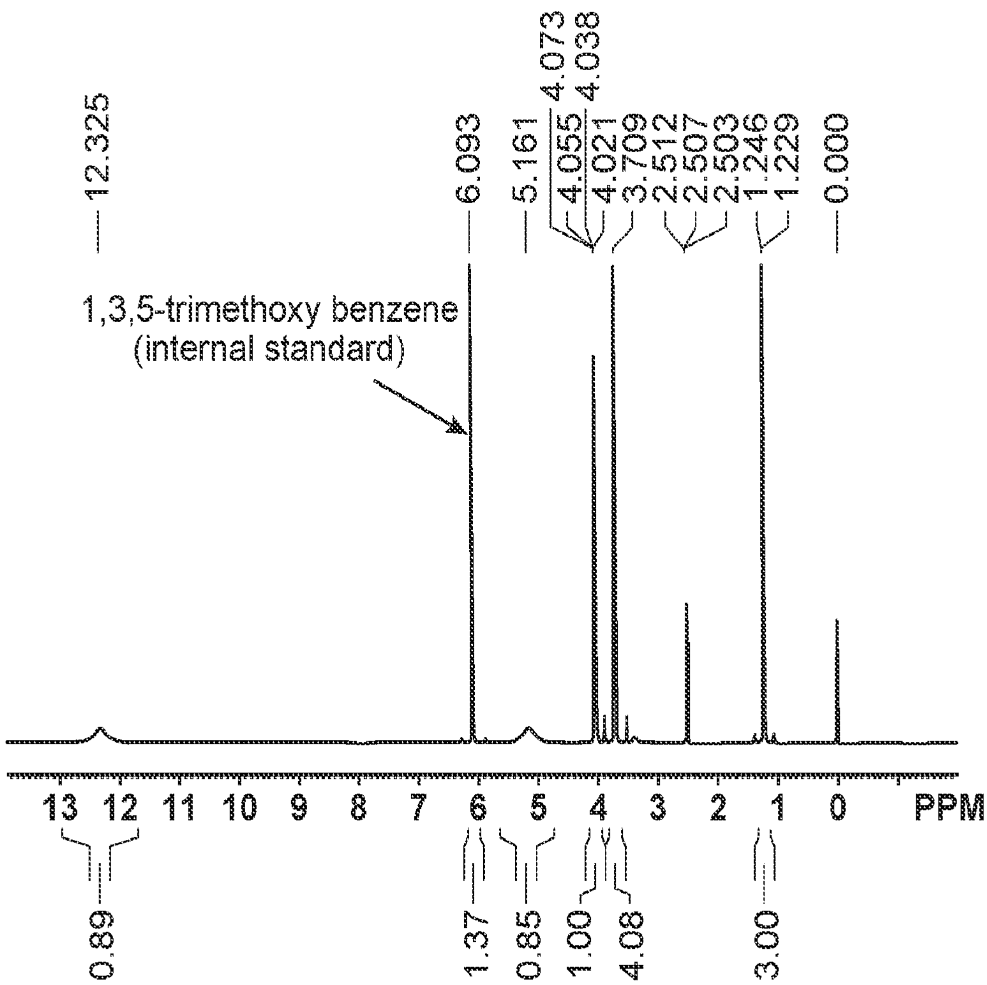
12.325
6.093
5.161
4.073
4.055
4.038
4.021
3.709
2.512
2.507
2.503
1.246
1.229
0.000
1,3,5-trimethoxy benzene
(internal standard)
13 12 11 10 9 8 7 6 5 4 3 2 1 0 PPM
0.89
1.37
0.85
1.00
4.08
3.00

FIG. 4

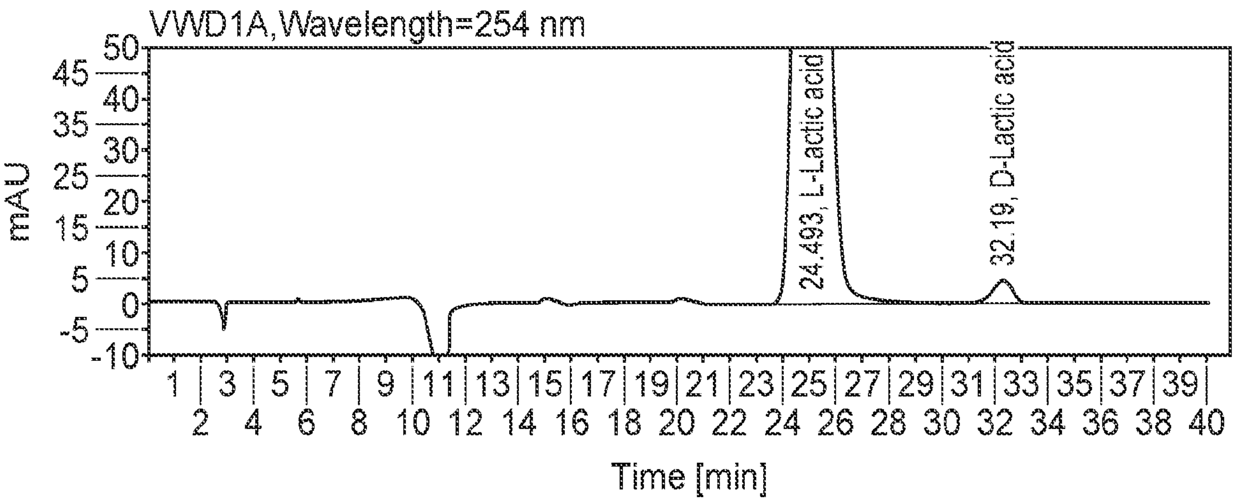
VWD1A,Wavelength=254 nm
mAU
50 45 40 35 30 25 20 15 10 5 0 -5 -10
24.493, L-Lactic acid
32.19, D-Lactic acid
1 3 5 7 9 11 13 15 17 19 21 23 25 27 29 31 33 35 37 39
2 4 6 8 10 12 14 16 18 20 22 24 26 28 30 32 34 36 38 40
Time [min]

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2232554 A
- EP 2015067258 W
- US 6630603 B
- EP 1317408 A
- US 20110319660 A
- WO 0125180 A1
- US 2331948 A
- WO 2015092420 A


### Non-patent literature cited in the description


- **PHILIPP, BABILAS** ; **ULRICH, KNIE** ; **CHRISTOPH, ABELS**. Cosmetic and dermatologic use of alpha hydroxy acids. *Journal of German Society of Dermatology*, 2012, vol. 10 (7), 488-49
- **KOMESU, A.** ; **OLIVEIRA, J. A. R. D.** ; **MARTINS, L. H. D. S.** ; **WOLF MACIEL, M. R.** ; **MACIEL FILHO, R.** Lactic acid production to purification: A review. *BioResources*, 2017, vol. 12 (2), 4364-4383
- Lactic Acid Market Size, Share & Trend Analysis Report. *Report ID: 978-1-68038-126-9*, 2019
- **BORSOOK, H.** ; **HUFFINAN, H.M.** ; **LIU, Y.-P.** The Preparation of Crystalline Lactic Acid. *J. Biol. Chem.*, 1933, vol. 102, 449
- **SIDNEY HSIN-HUAI CHOW**. Lactic Acid Review. *A Master's Thesis*, 1957